# EUROPEAN PATENT SPECIFICATION

(11) **EP 3 348 636 B1**
(45) Date of publication and mention of the grant of the patent: **01.12.2021**
(21) Application number: 16844434.7
(22) Date of filing: 08.09.2016
(51) Int. Cl.: C12N 9/22, C12N 9/78, C12N 15/09, C12N 15/10, C07K 14/245, C07K 19/00

(54) **METHOD FOR MODIFYING GENOME SEQUENCE THAT SPECIFICALLY CONVERTS NUCLEOBASE OF TARGETED DNA SEQUENCE, AND MOLECULAR COMPLEX USED IN SAID METHOD**
VERFAHREN ZUR MODIFIZIERUNG EINER GENOMSEQUENZ, DIE SPEZIFISCH DIE NUKLEOBASE EINER GEZIELTEN DNA-SEQUENZ UMWANDELT, UND IN DIESEM VERFAHREN VERWENDETER MOLEKULARER KOMPLEX
PROCÉDÉ DE MODIFICATION DE SÉQUENCE GÉNOMIQUE CONVERTISSANT SPÉCIFIQUEMENT UNE BASE NUCLÉIQUE D'UNE SÉQUENCE D'ADN CIBLÉE, ET COMPLEXE MOLÉCULAIRE UTILISÉ DANS LEDIT PROCÉDÉ

(30) Priority: 09.09.2015 JP 2015178023
(43) Date of publication of application: 18.07.2018
(73) Proprietor: National University Corporation Kobe University, Hyogo 657-8501 (JP)
(72) Inventor: NISHIDA, Keiji, Kobe-shi Hyogo 657-8501 (JP); KOJIMA, Satomi, Kobe-shi Hyogo 657-8501 (JP); KONDO, Akihiko, Kobe-shi Hyogo 657-8501 (JP)
(74) Representative: J A Kemp LLP
(86) International application number: PCT/JP2016/076448
(87) International publication number: WO 2017/043573

(56) References cited:
- WO-A1-2013/098244
- WO-A1-2014/071235
- WO-A1-2015/089406
- WO-A1-2015/089406
- WO-A2-2015/035136
- WO-A2-2015/035136
- JP-A- 2015 503 535
- US-A1- 2011 104 787
- US-A1- 2011 104 787
- MITCHELL R. O'CONNELL ET AL: "Programmable RNA recognition and cleavage by CRISPR/Cas9", NATURE, vol. 516, no. 7530, 28 September 2014 (2014-09-28), pages 263-266, XP055168138, ISSN: 0028-0836, DOI: 10.1038/nature13769
- SZYF MOSHE ET AL.: 'Maternal care, the epigenome and phenotypeic differences in behavior' REPRODUCTIVE TOXICOLOGY vol. 24, no. 1, July 2007, pages 9 - 19, XP027495178
- BOGDANOVE ADAM J. ET AL.: 'TAL Effectors: Customizable Proteins for DNA Targeting' SCIENCE vol. 333, no. 6501, 30 September 2011, pages 1843 - 1846, XP055152326
- MUSSOLINO CLAUDIO ET AL.: 'TALE nucleases: tailored genome engineering made easy' CURRENT OPINION IN BIOTECHNOLOGY vol. 23, no. 5, October 2012, pages 644 - 650, XP055071668
- LADA ARTEM G. ET AL.: 'Genome-Wide Mutation Avalanches Induced in Diploid Yeast Cells by a Base Analog or an APOBEC Deaminase' PLOS GENETICS vol. 9, no. ISSUE9, 01 September 2013, pages 1 - 17, XP055370984 DOI: 10.1371/JOURNAL.PGEN.1003736

### SEQUENCE LISTING

<110> NATIONAL UNIVERSITY CORPORATION KOBE UNIVERSITY
<120> Method of modifying genomic sequence comprising specifically converting nucleobase in targeted DNA sequence and molecular complex used therefor
<130> 092522
<150> JP 2015-178023
   <151> 2015-09-09
<160> 4
<170> PatentIn version 3.5
<210> 1
   <211> 624
   <212> DNA
   <213> Petromyzon marinus
<220>
   <221> CDS
   <222> (1)..(624)
<400> 1
   atg acc gac gct gag tac gtg aga atc cat gag aag ttg gac atc tac          48
   Met Thr Asp Ala Glu Tyr Val Arg Ile His Glu Lys Leu Asp Ile Tyr
   1               5                   10                  15
   acg ttt aag aaa cag ttt ttc aac aac aaa aaa tcc gtg tcg cat aga          96
   Thr Phe Lys Lys Gln Phe Phe Asn Asn Lys Lys Ser Val Ser His Arg
               20                  25                  30
   tgc tac gtt ctc ttt gaa tta aaa cga cgg ggt gaa cgt aga gcg tgt         144
   Cys Tyr Val Leu Phe Glu Leu Lys Arg Arg Gly Glu Arg Arg Ala Cys
           35                  40                  45
   ttt tgg ggc tat gct gtg aat aaa cca cag agc ggg aca gaa cgt ggc         192
   Phe Trp Gly Tyr Ala Val Asn Lys Pro Gln Ser Gly Thr Glu Arg Gly
       50                  55                  60
   att cac gcc gaa atc ttt agc att aga aaa gtc gaa gaa tac ctg cgc         240
   Ile His Ala Glu Ile Phe Ser Ile Arg Lys Val Glu Glu Tyr Leu Arg
   65                  70                  75                  80
   gac aac ccc gga caa ttc acg ata aat tgg tac tca tcc tgg agt cct         288
   Asp Asn Pro Gly Gln Phe Thr Ile Asn Trp Tyr Ser Ser Trp Ser Pro
                   85                  90                  95
   tgt gca gat tgc gct gaa aag atc tta gaa tgg tat aac cag gag ctg         336
   Cys Ala Asp Cys Ala Glu Lys Ile Leu Glu Trp Tyr Asn Gln Glu Leu
               100                 105                 110
   cgg ggg aac ggc cac act ttg aaa atc tgg gct tgc aaa ctc tat tac         384
   Arg Gly Asn Gly His Thr Leu Lys Ile Trp Ala Cys Lys Leu Tyr Tyr
           115                 120                 125
   gag aaa aat gcg agg aat caa att ggg ctg tgg aac ctc aga gat aac         432
   Glu Lys Asn Ala Arg Asn Gln Ile Gly Leu Trp Asn Leu Arg Asp Asn
       130                 135                 140
   ggg gtt ggg ttg aat gta atg gta agt gaa cac tac caa tgt tgc agg         480
   Gly Val Gly Leu Asn Val Met Val Ser Glu His Tyr Gln Cys Cys Arg
   145                 150                 155                 160
   aaa ata ttc atc caa tcg tcg cac aat caa ttg aat gag aat aga tgg         528
   Lys Ile Phe Ile Gln Ser Ser His Asn Gln Leu Asn Glu Asn Arg Trp
                   165                 170                 175
   ctt gag aag act ttg aag cga gct gaa aaa cga cgg agc gag ttg tcc         576
   Leu Glu Lys Thr Leu Lys Arg Ala Glu Lys Arg Arg Ser Glu Leu Ser
               180                 185                 190
   att atg att cag gta aaa ata ctc cac acc act aag agt cct gct gtt         624
   Ile Met Ile Gln Val Lys Ile Leu His Thr Thr Lys Ser Pro Ala Val
           195                 200                 205
<210> 2
   <211> 208
   <212> PRT
   <213> Petromyzon marinus
<400> 2
   Met Thr Asp Ala Glu Tyr Val Arg Ile His Glu Lys Leu Asp Ile Tyr
   1               5                   10                  15
   Thr Phe Lys Lys Gln Phe Phe Asn Asn Lys Lys Ser Val Ser His Arg
               20                  25                  30
   Cys Tyr Val Leu Phe Glu Leu Lys Arg Arg Gly Glu Arg Arg Ala Cys
           35                  40                  45
   Phe Trp Gly Tyr Ala Val Asn Lys Pro Gln Ser Gly Thr Glu Arg Gly
       50                  55                  60
   Ile His Ala Glu Ile Phe Ser Ile Arg Lys Val Glu Glu Tyr Leu Arg
   65                  70                  75                  80
   Asp Asn Pro Gly Gln Phe Thr Ile Asn Trp Tyr Ser Ser Trp Ser Pro
                   85                  90                  95
   Cys Ala Asp Cys Ala Glu Lys Ile Leu Glu Trp Tyr Asn Gln Glu Leu
               100                 105                 110
   Arg Gly Asn Gly His Thr Leu Lys Ile Trp Ala Cys Lys Leu Tyr Tyr
           115                 120                 125
   Glu Lys Asn Ala Arg Asn Gln Ile Gly Leu Trp Asn Leu Arg Asp Asn
       130                 135                 140
   Gly Val Gly Leu Asn Val Met Val Ser Glu His Tyr Gln Cys Cys Arg
   145                 150                 155                 160
   Lys Ile Phe Ile Gln Ser Ser His Asn Gln Leu Asn Glu Asn Arg Trp
                   165                 170                 175
   Leu Glu Lys Thr Leu Lys Arg Ala Glu Lys Arg Arg Ser Glu Leu Ser
               180                 185                 190
   Ile Met Ile Gln Val Lys Ile Leu His Thr Thr Lys Ser Pro Ala Val
           195                 200                 205
<210> 3
   <211> 21
   <212> DNA
   <213> Escherichia coli
<400> 3
   gagttccccg cgccagcggg g 21
<210> 4
   <211> 10821
   <212> DNA
   <213> Artificial Sequence
<220>
   <223> Expression vector carrying chimeric crRNA and Cascade-PmCDAl
<220>
   <221> misc_feature
   <222> (8490)..(8521)
   <223> n is a, c, g, or t
<400> 4
   gtcaatgccg agcgaaagcg agccgaaggg tagcatttac gttagataac cccctgatat      60
   gctccgacgc tttatataga aaagaagatt caactaggta aaatcttaat ataggttgag     120
   atgataaggt ttataaggaa tttgtttgtt ctaatttttc actcattttg ttctaatttc     180
   ttttaacaaa tgttcttttt tttttagaac agttatgata tagttagaat agtttaaaat     240
   aaggagtgag aaaaagatga aagaaagata tggaacagtc tataaaggct ctcagaggct     300
   catagacgaa gaaagtggag aagtcataga ggtagacaag ttataccgta aacaaacgtc     360
   tggtaacttc gtaaaggcat atatagtgca attaataagt atgttagata tgattggcgg     420
   aaaaaaactt aaaatcgtta actatatcct agataatgtc cacttaagta acaatacaat     480
   gatagctaca acaagagaaa tagcaaaagc tacaggaaca agtctacaaa cagtaataac     540
   aacacttaaa atcttagaag aaggaaatat tataaaaaga aaaactggag tattaatgtt     600
   aaaccctgaa ctactaatga gaggcgacga ccaaaaacaa aaatacctct tactcgaatt     660
   tgggaacttt gagcaagagg caaatgaaat agattgacct cccaataaca ccacgtagtt     720
   attgggaggt caatctatga aatgcgatta agctttttct aattcacata agcgtgcagg     780
   tttaaagtac ataaaaaata taatgaaaaa aagcatcatt atactaacgt tataccaaca      840
   ttatactctc attatactaa ttgcttattc caatttccta ttggttggaa ccaacaggcg      900
   ttagtgtgtt gttgagttgg tactttcatg ggattaatcc catgaaaccc ccaaccaact      960
   cgccaaagct ttggctaaca cacacgccat tccaaccaat agttttctcg gcataaagcc     1020
   atgctctgac gcttaaatgc actaatgcct taaaaaaaca ttaaagtcta acacactaga     1080
   cttatttact tcgtaattaa gtcgttaaac cgtgtgctct acgaccaaaa gtataaaacc     1140
   tttaagaact ttcttttttc ttgtaaaaaa agaaactaga taaatctctc atatctttta     1200
   ttcaataatc gcatcagatt gcagtataaa tttaacgatc actcatcatg ttcatattta     1260
   tcagagctcg tgctataatt atactaattt tataaggagg aaaaaataaa gagggttata     1320
   atgaacgaga aaaatataaa acacagtcaa aactttatta cttcaaaaca taatatagat     1380
   aaaataatga caaatataag attaaatgaa catgataata tctttgaaat cggctcagga     1440
   aaagggcatt ttacccttga attagtacag aggtgtaatt tcgtaactgc cattgaaata     1500
   gaccataaat tatgcaaaac tacagaaaat aaacttgttg atcacgataa tttccaagtt     1560
   ttaaacaagg atatattgca gtttaaattt cctaaaaacc aatcctataa aatatttggt     1620
   aatatacctt ataacataag tacggatata atacgcaaaa ttgtttttga tagtatagct     1680
   gatgagattt atttaatcgt ggaatacggg tttgctaaaa gattattaaa tacaaaacgc     1740
   tcattggcat tatttttaat ggcagaagtt gatatttcta tattaagtat ggttccaaga     1800
   gaatattttc atcctaaacc taaagtgaat agctcactta tcagattaaa tagaaaaaaa     1860
   tcaagaatat cacacaaaga taaacagaag tataattatt tcgttatgaa atgggttaac     1920
   aaagaataca agaaaatatt tacaaaaaat caatttaaca attccttaaa acatgcagga     1980
   attgacgatt taaacaatat tagctttgaa caattcttat ctcttttcaa tagctataaa     2040
   ttatttaata agtaagttaa gggatgcata aactgcatcc cttaacttgt ttttcgtgta     2100
   cctatttttt gtgaatcgcc attcgccatt caggctgcgc aactgttggg aagggcgatc     2160
   ggtgcgggcc tcttcgctat tacgccagct ggcgaaaggg ggatgtgctg caaggcgatt     2220
   aagttgggta acgccagggt tttcccagtc acgacgttgt aaaacgacgg ccagtgaatt     2280
   cgagctcggt acccggccgc aaacaacaga taaaacgaaa ggcccagtct ttcgactgag     2340
   cctttcgttt tatttgatgc ctgtcaagta acagcaggac tcttagtggt gtggagtatt     2400
   tttacctgaa tcataatgga caactcgctc cgtcgttttt cagctcgctt caaagtcttc     2460
   tcaagccatc tattctcatt caattgattg tgcgacgatt ggatgaatat tttcctgcaa     2520
   cattggtagt gttcacttac cattacattc aacccaaccc cgttatctct gaggttccac     2580
   agcccaattt gattcctcgc atttttctcg taatagagtt tgcaagccca gattttcaaa     2640
   gtgtggccgt tcccccgcag ctcctggtta taccattcta agatcttttc agcgcaatct 2700
   gcacaaggac tccaggatga gtaccaattt atcgtgaatt gtccggggtt gtcgcgcagg 2760
   tattcttcga cttttctaat gctaaagatt tcggcgtgaa tgccacgttc tgtcccgctc 2820
   tgtggtttat tcacagcata gccccaaaaa cacgctctac gttcaccccg tcgttttaat 2880
   tcaaagagaa cgtagcatct atgcgacacg gattttttgt tgttgaaaaa ctgtttctta 2940
   aacgtgtaga tgtccaactt ctcatggatt ctcacgtact cagcgtcggt catcctagac 3000
   ttatcgtcat cgtctttgta atcaatatca tgatccttgt agtctccgtc gtggtcctta 3060
   tagtctccgg actcgagtcc cagtggagcc aaagatagca agccacatcc catcgattta 3120
   gctggcccaa taccttgctg tacaagatct attaacgctg gcgcgtcgtt gatggtgagc 3180
   acaccttcaa agcaaaccgt ttggatcttt ccacttttac catcaccaga aaaatactgt 3240
   ggccgttccg atatgggatg cacatcttca acgcgcgccg cattgcccaa tttacgttgc 3300
   aaccacgcga tttgttctgc ttcttttatt aacggaaccc gacagcgttt aatattccct 3360
   ttactgtcca ggcgcttttg attgtcgaga atagttttga tcggatttgc ccgaagccga 3420
   aaatagagtg gaacaccaac ctgaagttga aattcaacct gtttagtttt aatgactgtc 3480
   gcaacggcag ttgaaacagg catttgcgct gactgcaata aaacatgaca gccttctggt 3540
   gtgtttcgct tctcaacatg aaaaagaaaa tcacgagcag catccggtct gtttggaaat 3600
   aaatgccata atccctggtg aagttggtaa agatccctgc tccaggccct ggcaatgatg 3660
   actttactga gatacatcca tacctccttt aatcacatac cattctcggg aagcaaattg 3720
   tcgaggcaag gtgatcatcg gttcgtcgcg cgccgtaaat tttaaatgat gccctgtaac 3780
   tgattcctca ctatatatat cgccgccaac gggctcataa tttaatagcg ccttctgagg 3840
   atccgatgcc tgacatgtcc ccaaaaaaag cgggtgtgtt agtgggcaac ttctccgccc 3900
   caggtaaggt gtataccgag gctttaatac tgctttttca agttctgaga taaccatcgt 3960
   tgcatggggt gttaaccaga gagcgacggt aaaggaggca tcacataaat attcgcgcca 4020
   tgtttgaatc gtttcatgac ttttcaaacc acggtaatct tctcgcgctc caaggactgt 4080
   atggtaatca cgcaaccccg ttacagacac acgacgatcg tcaagaatga gttcatcgca 4140
   gcgcactgca aattgcacac tctctgataa cgcctgtaat gaagaagtat catcacgttg 4200
   gatcccaaga caagccccga gtagccctaa taacccgctt cgggtcggaa atcttccggt 4260
   aggtcgcgtt ccttcaaagg tcggctgccc ccaggcttgc attggcccag caagccgcaa 4320
   gatcaaataa gatctcatgt tcacgcctcg ccattattac gaacccagga ttttaactgt 4380
   tctaaagtag gcatttgttt aacttgagca gtaattgggt ctacatcaga taagctgaat 4440
   tgcgcagcag ctccgttcag accatatcca ttggcaacgc gatcccaata ttgattaaac 4500
   gcctgtatag acggttgcaa aaagccatct ttcgctttaa ccgctttttc aaaagcattt 4560
   gccatagaaa gtggcatatc ggagaaatta accattacca tatccgcagg gttaaaagcg 4620
   gcataagtac gctgttttgc tccagggacc tctgttgcca gcatatgaac aacatgggtt 4680
   gcaatttcca gagcctgctc cctggaggca ccacctaaat tttcctgaag ttgagcgagg 4740
   ttaatgttgg cataacgata aaaaacaccc gatgaaaatt cctgagttcc cagatgtgca 4800
   gaaccttgtt cctgtaaatc atctacagcg gtgaaccagt caatatcaga atcaacctga 4860
   tgagtagtga tcgcatgcgc aatggacatt gcaccatcaa cttttcccaa ctcagtcatc 4920
   atgccgctgg ttgccattct tccactaagc gcaatatcaa caccctgctg taaattcaca 4980
   cgtatggcgg caatatcttc cttaagaact ttgagcagct ttttatcatc cagattatca 5040
   gcctctgctt ttgcaacctg ctcacagaac caggctattt ctcccacaac ccagggagta 5100
   accgcatcgg cagaaatctt ttcggcttca tcaactgatt taccggagag cagcgctaat 5160
   gtcttatcga tgattttttg gtcaaaacgt tcaccaagtt tttgccgaag aacatcacgt 5220
   aattgtgcaa gatgaatggt tctgagactg gattcaccaa tattttgtgc gtaataacca 5280
   cttttacgca tcgcacgttt aaggctttga cttgaaattc ttactcgtct tttgccgccg 5340
   aaaatagcgt ctttctgcat gttcatatcg tcgcggttca gacatgaagg gctgtgagag 5400
   atcagaacat gaatattgat aaagttagac atagaaaggt ttcctcctag aactaggtta 5460
   cgcatttttg tttgtggtca atacaaaatc ttccagaagt tgctggcgtt cgcgctttcc 5520
   ccaccaggtc aacatcctgg ccattaatgg ccagtcaagt acgggttcgg cgtgagtaag 5580
   taatcgacgt aactggacca tatcggctgt tctgtcagcc cgaattaatt gaaagatacg 5640
   gcgctcgtta attcttccac tattggctaa agctctgccc aacgagatac ctgttgtttg 5700
   ctccgatttt ttgtcctgat gtcggatgac attctttcct gcgctcaggc aaaacaccat 5760
   gcgcaaaaga gcctgctggt gacgtgggtt ttcccaacca aaaggttgca ccagcctata 5820
   aaacgcaggg atatcgcgta attcatcagg ttctgaaaca cgtctaattt gcgcacatga 5880
   tccattatcc agttgttgcc aggctcgata taaagccatt gcatcaattt catcagccat 5940
   ttgatggccc tccttgcggt tttaactccc gtaaatgttt gtatagcgtg gcgcgggcaa 6000
   gcgctaatgt gcttattaat ttaggatgat gtgcataggg agctacagat tgattaaata 6060
   gcatttcaca caattgatga agtttgtctc gtaaatcagc tattacctca tcagcctggg 6120
   aaaaattaac attcgccagt acatcgggaa ttaataattc actctgtcga tagaaatgcc 6180
   tttctgcagt ctcatgaaca gagactccgg cccctttgaa gtctttattt ttaaaccctt 6240
   ctgcaaaggt atataacgcc ttgcgtaagg ctgttttata tcccaaacca acagtcacta 6300
   tttcgtttat cacattgccg tattgttgcc acccctgatt aaacatcaac acatcatgac 6360
   gccgttcaag aatagatgct tgattattac gatatccccc cataatcaat tcaagaggac 6420
   tttgcggcgc aatatttctg aattgattca caaccgccgc cacgcgattt ccattttcat 6480
   tttgaataat cttatctacc acaactcggc tgatttgtgt ccatgatggt gcggaggtgg 6540
   tgaaagcaag aaatttttcc tcaacctccc ctttcttgac tgttaccaga caaggggaat 6600
   gcggatgggg ccatagccca ttaactgtaa aggtaaattt ttccttaaga aaaccggtat 6660
   aacgcaaatt gctttcctgt ccacagcaag aacatttacc aatcccaatg ggatcgcata 6720
   attcaatatg cgctggttgc cagaatagac cacggacaaa cccaattgac gaagcaggta 6780
   tagactcatt ggacttgata ggtttaatcc aggtaggttg gttttccgta tgtgattcat 6840
   taggaaattg tttttgaaga cgaggtaatg tgaggacatt gagtaacacc gttgaacgaa 6900
   gatcgatccc acgtacgaac gttgttacag gtgttcctcc acgtaaaccg cttttaaaac 6960
   caccaccaaa acctggtgcc tgattcgcct ggttgaataa cgcaatcgca gtgcatccac 7020
   cacataatgc ttcaccctgc cccggttgat tgacaaatgc acaattcgtc gcgccgctta 7080
   ccccagccaa cagtttttcc attggagtca catcatttgc tttgacacct ttggtctgca 7140
   taaagggatg ttctgcgtga ttaaggtaga acatatctat ccacggcgcg atgagttgtt 7200
   gaaactcatc ttcagtgagc ggattcatta tgcgatgtcg aaattcaacg tcatcttttg 7260
   ccggggcgat aatttgccca atgcaaacca gcagtgctaa agcggccagt tccatatcgt 7320
   cacggggcaa acttaatcgc cactgatctc tactgcagta tagcgattgc agatttatga 7380
   tttggacttt ccccccgttt cgcgggcgta cagggatcca gttatcaata agcaaattca 7440
   tttgttctcc ttcatatggg ccctgcaacc attatcaccg ccagaggtaa aatagtcaac 7500
   acgcacggtg ttagatattt atcccttgcg gtgatagatt taacgttaag gaggtatgta 7560
   tgagcacaaa aaagaaacca ttaacacaag agcagcttga ggacgcacgt cgccttaaag 7620
   caatttatga aaaaaagaaa aatgaacttg gcttatccca ggaatctgtc gcagacaaga 7680
   tggggatggg gcagtcaggc gttggtgctt tatttaatgg catcaatgca ttaaatgctt 7740
   ataacgccgc attgcttaca aaaattctca aagttagcgt tgaagaattt agcccttcaa 7800
   tcgccagaga aatctacgag atgtatgaag cggttagtat gcagccgtca cttagaagtg 7860
   agtatgagta ccctgttttt tctcatgttc aggcagggat gttctcacct aagcttagaa 7920
   cctttaccaa aggtgatgcg gagagatggg taagcacaac caaaaaagcc agtgattctg 7980
   cattctggct tgaggttgaa ggtaattcca tgaccgcacc aacaggctcc aagccaagct 8040
   ttcctgacgg aatgttaatt ctcgttgacc ctgagcaggc tgttgagcca ggtgatttct 8100
   gcatagccag acttgggggt gatgagttta ccttcaagaa actgatcagg gatagcggtc 8160
   aggtgttttt acaaccacta aacccacagt acccaatgat cccatgcaat gagagttgtt 8220
   ccgttgtggg gaaagttatc gctagtcagt ggcctgaaga gacgtttggc tgatcggcaa 8280
   ggtgttctgg tcggcgcata gctgataaca attgagcaag aatcttcatc gataaccatc 8340
   tgcggtgata aattatctct ggcggtgttg acataaatac cactggcggt gataattctg 8400
   cagtagagag ctagcgagtt ccccgcgcca gcggggataa accgtgagac catggtctca 8460
   gagttccccg cgccagcggg gataaaccgn nnnnnnnnnn nnnnnnnnnn nnnnnnnnnn 8520
   ngagttcccc gcgccagcgg ggataaaccg tctagaaaaa accccctcaa gacccgttta 8580
   gaggccccaa ggggttatgc tactagaccg caaaaatcag cgcgcaaata cgcatactgt 8640
   tatctggctt ttagtaagcc ggatccacgc gtttacgccc cgccctgcca ctcatcgcag 8700
   tactgttgta attcattaag cattctgccg acatggaagc catcacagac ggcatgatga 8760
   acctgaatcg ccagcggcat cagcaccttg tcgccttgcg tataatattt gcccatggtg 8820
   aaaacggggg cgaagaagtt gtccatattg gccacgttta aatcaaaact ggtgaaactc 8880
   acccagggat tggctgagac gaaaaacata ttctcaataa accctttagg gaaataggcc 8940
   aggttttcac cgtaacacgc cacatcttgc gaatatatgt gtagaaactg ccggaaatcg 9000
   tcgtggtatt cactccagag cgatgaaaac gtttcagttt gctcatggaa aacggtgtaa 9060
   caagggtgaa cactatccca tatcaccagc tcaccgtctt tcattgccat acggaattcc 9120
   ggatgagcat tcatcaggcg ggcaagaatg tgaataaagg ccggataaaa cttgtgctta 9180
   tttttcttta cggtctttaa aaaggccgta atatccagct gaacggtctg gttataggta 9240
   cattgagcaa ctgactgaaa tgcctcaaaa tgttctttac gatgccattg ggatatatca 9300
   acggtggtat atccagtgat ttttttctcc attttagctt ccttagctcc tgaaaatctc 9360
   gccggatcct aactcaaaat ccacacatta tacgagccgg aagcataaag tgtaaagcct 9420
   ggggtgccta atgcggcccc tctagctacc ggttttggtc atgagattat ctctgtgcat 9480
   atggacagtt ttccctttga tatgtaacgg tgaacagttg ttctactttt gtttgttagt 9540
   cttgatgctt cactgataga tacaagagcc ataagaacct cagatccttc cgtatttagc 9600
   cagtatgttc tctagtgtgg ttcgttgttt ttgcgtgagc catgagaacg aaccattgag 9660
   atcatgctta ctttgcatgt cactcaaaaa ttttgcctca aaactggtga gctgaatttt 9720
   tgcagttaaa gcatcgtgta gtgtttttct tagtccgtta tgtaggtagg aatctgatgt 9780
   aatggttgtt ggtattttgt caccattcat ttttatctgg ttgttctcaa gttcggttac 9840
   gagatccatt tgtctatcta gttcaacttg gaaaatcaac gtatcagtcg ggcggcctcg 9900
   cttatcaacc accaatttca tattgctgta agtgtttaaa tctttactta ttggtttcaa 9960
   aacccattgg ttaagccttt taaactcatg gtagttattt tcaagcatta acatgaactt 10020
   aaattcatca aggctaatct ctatatttgc cttgtgagtt ttcttttgtg ttagttcttt 10080
   taataaccac tcataaatcc tcatagagta tttgttttca aaagacttaa catgttccag 10140
   attatatttt atgaattttt ttaactggaa aagataaggc aatatctctt cactaaaaac 10200
   taattctaat ttttcgcttg agaacttggc atagtttgtc cactggaaaa tctcaaagcc    10260
   tttaaccaaa ggattcctga tttccacagt tctcgtcatc agctctctgg ttgctttagc    10320
   taatacacca taagcatttt ccctactgat gttcatcatc tgaacgtatt ggttataagt    10380
   gaacgatacc gtccgttctt tcattgtagg gttttcaatc gtggggttga gtagtgccac    10440
   acagcataaa attagcttgg tttcatgctc cgttaagtca tagcgactaa tcgctagttc    10500
   atttgctttg aaaacaacta attcagacat acatctcaat tggtctaggt gattttaatc    10560
   actataccaa ttgagatggg ctagtcaatg ataattacta gtccttttcc tttgagttgt    10620
   gggtatctgt aaattctgct agacctttgc tggaaaactt gtaaattctg ctagaccctc    10680
   tgtaaattcc gctagacctt tgtgtgtttt ttttgtttat attcaagtgg ttataattta    10740
   tagaataaag aaagaataaa aaaagataaa aagaatagat cccagccctg tgtataactc    10800
   actactttag tcagttccga c                                              10821

## Description

### [Technical Field]

The present invention relates to a method for modifying a genomic sequence without cleaving a double stranded DNA or inserting a foreign DNA fragment and enabling modification of a nucleic acid base within a particular region of the genome, and to a complex of a nucleic acid sequence-recognizing module and a deaminase to be used therefor.

### [Background Art]

In recent years, genome editing is attracting attention as a technique for modifying the object gene and genome region in various species. Conventionally, as a method of genome editing, a method utilizing an artificial nuclease comprising a molecule having a sequence-independent DNA cleavage ability and a molecule having a sequence recognition ability in combination has been proposed (non-patent document 1).

For example, a method of performing recombination at a target gene locus in DNA in a plant cell or insect cell as a host, by using a zinc finger nuclease (ZFN) wherein a zinc finger DNA binding domain and a non-specific DNA cleavage domain are linked (patent document 1), a method of cleaving or modifying a target gene in a particular nucleotide sequence or a site adjacent thereto by using TALEN wherein a transcription activator-like (TAL) effector which is a DNA binding module that the plant pathogenic bacteria Xanthomonas has, and a DNA endonuclease are linked (patent document 2), a method utilizing CRISPR-Cas9 system wherein DNA sequence CRISPR (Clustered Regularly interspaced short palindromic repeats) that functions in an acquired immune system possessed by eubacterium and archaebacterium, and nuclease Cas (CRISPR-associated) protein family having an important function along with CRISPR are combined (patent document 3) and the like have been reported. Furthermore, a method of cleaving a target gene in the vicinity of a particular sequence, by using artificial nuclease wherein a PPR protein constituted to recognize a particular nucleotide sequence by a continuation of PPR motifs each consisting of 35 amino acids and recognizing one nucleic acid base, and nuclease are linked (patent document 4) has also been reported.

The genome editing techniques proposed heretofore basically presuppose double stranded DNA breaks (DSB). However, since they include unexpected genome modifications, side effects such as strong cytotoxicity, chromosomal rearrangement and the like occur, and they have common problems of impaired reliability in gene therapy, extremely small number of surviving cells by nucleotide modification, and difficulty in genetic modification itself in primate ovum and unicellular microorganisms.

As a genome editing technique unaccompanied by DSB, an artificial enzyme obtained by combining a molecule having a sequence recognition ability such as zinc finger (ZF) motif and the like and deaminase that converts an amino group of a nucleic acid base to a carbonyl group has been proposed (patent document 5). However, experimental proof does not exist, and it is not clear whether genetic modification is at all possible, not to mention mutation introduction efficiency thereof. Indeed, as a system similarly unaccompanied by genome cleavage, a method using DNA glycosylase that catalyzes deamination reaction on the DNA strand shows an extremely low mutation introduction efficiency even when a mutant yeast with an attenuated DNA repair mechanism is used as a host (non-patent document 2), and practicalization for gene therapy, molecular breeding of useful organisms and the like is further away as the situation stands. WO 2013/098244 (patent document 6) discloses modified cascade ribonucleoproteins and uses thereof. WO 2015/089406 (patent document 7) discloses CAS variants for gene editing, such as fusion proteins of CAS9 and nucleic acid editing enzymes. WO 2015/035136 (patent document 8) discloses systems for delivery of functional effector proteins using cationic lipids and cationic polymers.

### [Document List]

### [Patent documents]

patent document 1: JP-B-4968498
patent document 2: National Publication of International Patent Application No. 2013-513389
patent document 3: National Publication of International Patent Application No. 2010-519929
patent document 4: JP-A-2013-128413
patent document 5: US-A-2011/0104787
patent document 6: WO 2013/098244
patent document 7: WO 2015/089406
patent document 8: WO 2015/035136

### [non-patent document]

non-patent document 1: Kelvin M Esvelt, Harris H Wang (2013) Genome-scale engineering for systems and synthetic biology, Molecular Systems Biology 9: 641
non-patent document 2: Prashant Mali, Kevin M Esvelt, George M Church (2013) Nucleic Acids Res. 41: e99

### [SUMMARY OF THE INVENTION]

### [Problems to be Solved by the Invention]

Therefore, an object of the present invention is to provide a novel method of genome editing to convert a nucleic acid base in a particular sequence of a gene without DSB or insertion of a foreign DNA fragment, and a complex of a nucleic acid sequence-recognizing module and a deaminase therefor.

### [Means of Solving the Problems]

The present inventors have already succeeded in efficiently modifying, without accompanying DSB, genomic sequence in a region containing a particular DNA sequence, by using a Type II CRISPR-Cas system having mutant Cas9, in which cleavage ability of one or both strands of a double stranded DNA has been inactivated, as a nucleic acid sequence-recognizing module and deaminase as a nucleic acid base converting enzyme (WO 2015/133554). However, since Cas protein recognizes and binds to a sequence called protospacer adjacent motif (PAM) on a DNA strand, the mutation introduction site is limited by the presence or otherwise of PAM. Cas9 (SpCas9) derived from Streptococcus pyogenes in the Type II CRISPR-Cas system currently used frequently for genome editing recognizes NGG (N is any base) as PAM. It has also been clarified by the research of the present inventors that the nucleotide sequence to be targeted in Type II CRISPR (i.e., nucleotide sequence on DNA strand complementary to guide RNA) is 5' upstream 18 - 25 nucleotides of PAM in length and, despite its length, base conversion most frequently occurs at the positions of 2 - 5 nucleotides from the 5'-end of the target nucleotide sequence.

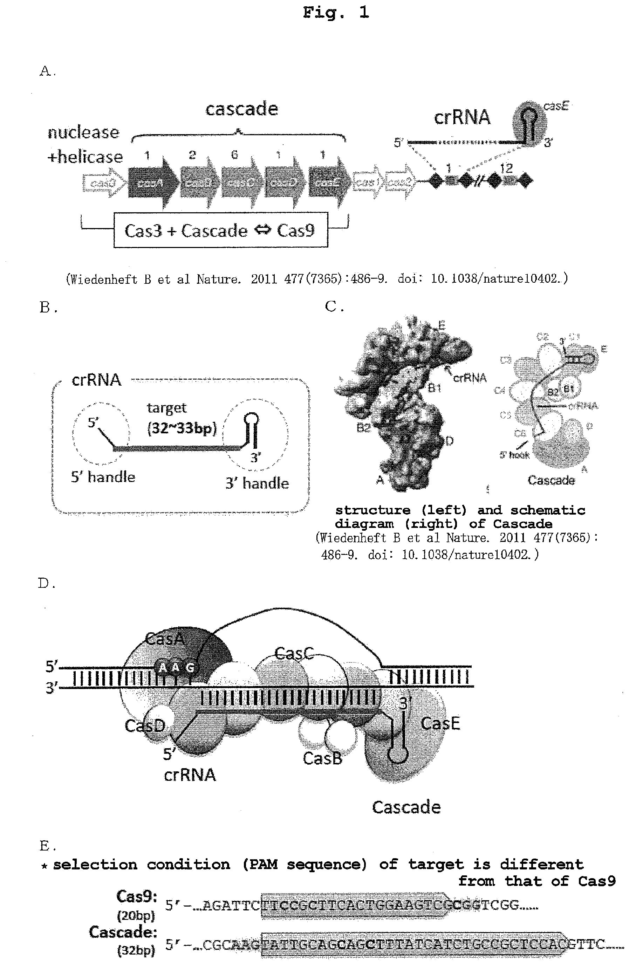

On the other hand, in the Type I-E CRISPR-Cas system of Escherichia coli and the like, it is known that ATG, AAG, AGG or GAG adjacent to the 5'-side of the target nucleotide sequence with 32 - 33 nucleotide length is recognized as PAM sequence (see Fig. 1; PAM sequence shows the case of AAG in Fig. 1D and E), and other Type I CRISPR-Cas systems also recognize specific PAM sequences of 2 or 3 bases. Therefore, if Type I CRISPR-Cas system can be used as a nucleic acid sequence-recognizing module, base conversion can be performed at a site where introduction of mutation is difficult for Cas9 due to the restriction of PAM sequence and introduction site of mutation.

In the Type II CRISPR-Cas system, a nucleic acid sequence-recognizing module could be constituted only from a Chimeric RNA of crRNA complementary to the target nucleotide sequence and trans-acting crRNA (tracrRNA) for recruiting Cas 9, and Cas9. However, for example, in the Type I-E CRISPR-Cas system of Escherichia coli, a complicated ribonucleoprotein complex composed of 5 kinds of Cas proteins (CasA, CasB, CasC, CasD and CasE) presenting crRNA, which is called CRISPR-associated complex for antiviral defense (Cascade), recognizes the target nucleotide sequence and the PAM sequence. Combined with Cas3 having nuclease and helicase activities, the complex exhibits a DNA recognition and cleavage function similar to that of Cas9 in the Type II CRISPR-Cas system (see Fig. 1). Due to such complicated constitution, the Type I CRISPR-Cas system has hardly been utilized even for genome editing technique as an artificial nuclease.

The present inventors prepared a DNA encoding genomic specific CRISPR-RNA in which 5' handle and 3' handle necessary for correct display in Cascade are linked at both ends of a sequence complementary to the target strand of the target nucleotide sequence of the rpoB gene which is an essential gene of Escherichia coli (crRNA). On the other hand, they isolated casA - casE gene group necessary for the constitution of Cascade from Cas operon of Escherichia coli, linked a deaminase gene thereto to produce a DNA, and introduced these DNAs into a host Escherichia coli containing a gene to be modified. As a result, they successfully introduced mutation into the target nucleotide sequence of the gene of interest and the vicinity thereof, without accompanying cleavage of the genome DNA. In addition, it was found that cytosine at 32 - 44 bases downstream from the PAM sequence is mainly edited when the length of the target nucleotide sequence is set to 32 nucleotides, unlike the use of Cas9.

The present inventors have conducted further studies based on these findings and completed the present invention.

That is, the present invention is as described below.
[1] A non-therapeutic method of modifying a targeted site of a double stranded DNA in a host cell, the method comprising introducing
   (a) a DNA encoding a crRNA comprising a sequence complementary to a target strand of a target nucleotide sequence in the given double stranded DNA, and
   (b) a DNA encoding a protein group constituting Cascade and a deaminase, in which the deaminase is constituted in a form capable of forming a complex with CasE in the protein group into the host cell to convert one or more nucleotides in the targeted site to other one or more nucleotides without cleaving the double stranded DNA in the targeted site, wherein the complex lacks Cas3, Cas1, Cas2 and Cas4, and wherein the aforementioned protein group constituting Cascade comprises CasA, CasB, CasC, CasD and CasE, and wherein the method is not a process for modifying the germ line genetic identity of human beings.
[2] The method of the above-mentioned [1], wherein the aforementioned Cascade is derived from Escherichia coli.
[3] The method of the above-mentioned [1] or [2], wherein the aforementioned deaminase is cytidine deaminase.
[4] The method of any of the above-mentioned [1] to [3], wherein the aforementioned host cell is a prokaryotic cell.
[5] The method of any of the above-mentioned [1] to [4], comprising a step of introducing an expression vector comprising the DNAs of the aforementioned (a) and (b) in a form capable of controlling an expression period into the aforementioned host cell, and inducing expression of the DNAs for a period necessary for fixing the modification of the targeted site in the double stranded DNA.
[6] The method of the above-mentioned [5], wherein the target nucleotide sequence in the double stranded DNA is present in a gene essential for the aforementioned host cell.
[7] A nucleic acid-modifying enzyme complex for modifying a targeted site of a double stranded DNA in a host cell, the complex comprising
   (a) a crRNA comprising a sequence complementary to a target strand of a target nucleotide sequence in the given double stranded DNA, and
   (b) a nucleic acid-modifying enzyme complex comprising a protein group constituting Cascade, and a deaminase that has formed a complex with CasE in the protein group, wherein the complex lacks Cas3, Cas1, Cas2 and Cas4, and wherein the aforementioned protein group constituting Cascade comprises CasA, CasB, CasC, CasD and CasE.
[8] The nucleic acid-modifying enzyme complex according to the above-mentioned [7], wherein the Cascade is derived from Escherichia coli.
[9] The nucleic acid-modifying enzyme complex according to the above-mentioned [7] or [8], wherein the deaminase is cytidine deaminase.
[10] A DNA encoding the nucleic acid-modifying enzyme complex of any of the above-mentioned [7] to [9].

### [Effect of the Invention]

The genome editing technique of the present invention does not accompany insertion of a foreign DNA or cleavage of double stranded DNA. Therefore, it is superior in safety and has no small possibility of becoming a solution to the cases associated with biological or legal disputes of gene recombination in conventional methods. In addition, using Cascade as a nucleic acid sequence-recognizing module, PAM sequence, which is different from Cas9, can be utilized and a mutation is introduced highly frequently into a different site, which expands the choice of the site into which a mutation can be introduced in the target gene.

### [Brief Description of the Drawings]

Fig. 1 schematically shows cas operon on the Escherichia coli genome and CRISPR gene locus (A), structure of crRNA (B), structure of Cascade (C), R-loop structure produced when Cascade recognizes and binds to the target nucleotide sequence (D), and comparison of the target nucleotide sequences and PAM sequences of Cas9 and Cascade (E).
Fig. 2 shows the constitution of respective subtypes (I-A to I-F) of the cas gene group in the Type I CRISPR-Cas system.
Fig. 3 is a schematic showing of the structure (top) of the major part of the representative vector used in the present invention, and genetic modification (bottom) by the nucleic acid-modifying enzyme complex of the present invention produced from the vector.
Fig. 4 relates to a physical map and the detailed sequence information of the vector used in the Examples.
Fig. 5-1 shows the sequencing results of the target nucleotide sequence and the vicinity thereof in the rifampicin resistant colony obtained by the modification of rpoB gene by using a Cascade-deaminase complex. Target 3r and target 5r were used.
Fig. 5-2 shows the sequencing results of the target nucleotide sequence and the vicinity thereof in the rifampicin resistant colony obtained by the modification of rpoB gene by using a Cascade-deaminase complex. Target 4r was used.

### [Description of Embodiments]

The present invention provides a non-therapeutic method of modifying a targeted site of a double stranded DNA by converting the target nucleotide sequence and nucleotides in the vicinity thereof in the double stranded DNA to other nucleotides, without cleaving the double stranded DNA to be modified in the host cell (hereinafter to be also referred to as "the method of the present invention"). The method of the present invention is not a process for modifying the germ line genetic identity of human beings. The method characteristically contains a step of contacting a complex wherein a nucleic acid sequence-recognizing module that specifically binds to the target nucleotide sequence in the double stranded DNA in the host cell and a deaminase are bonded with the double stranded DNA to convert the targeted site, i.e., the target nucleotide sequence and nucleotides in the vicinity thereof, to other nucleotides.

In the present invention, the "modification" of a double stranded DNA means that a nucleotide (e.g., dC) on a DNA strand is converted to other nucleotide (e.g., dT, dA or dG). While the double stranded DNA to be modified is not particularly limited as long as it is a double stranded DNA present in the host cell, it is preferably a genomic DNA. The "targeted site" of a double stranded DNA means the whole or partial "target nucleotide sequence", which a nucleic acid sequence-recognizing module specifically recognizes and binds to, or the vicinity of the target nucleotide sequence (one or both of 5' upstream and 3' downstream).

In the present invention, the "nucleic acid sequence-recognizing module" means a molecule or molecule complex having an ability to specifically recognize and bind to a particular nucleotide sequence (i.e., target nucleotide sequence) on a DNA strand. Binding of the nucleic acid sequence-recognizing module to a target nucleotide sequence enables a deaminase linked to the module to specifically act on a targeted site of a double stranded DNA.

In the present disclosure, the "nucleic acid base converting enzyme" means an enzyme capable of converting a target nucleotide to other nucleotide by catalyzing a reaction for converting a substituent on a purine or pyrimidine ring on a DNA base to other group or atom, without cleaving the DNA strand.

In the present invention, the "nucleic acid-modifying enzyme complex" means a molecule complex comprising a complex wherein the above-mentioned nucleic acid sequence-recognizing module and a deaminase are linked, which molecule complex is imparted with a particular nucleotide sequence recognition ability, and deaminase activity. The "complex" here encompasses not only one constituted of multiple molecules, but also one having a nucleic acid sequence-recognizing module and deaminase in a single molecule, like a fusion protein.

The deaminase to be used in the present invention is not particularly limited as long as it can catalyze the above-mentioned reaction, and examples thereof include deaminase belonging to the nucleic acid/nucleotide deaminase superfamily, which catalyzes a deamination reaction that converts an amino group to a carbonyl group. Preferable examples thereof include cytidine deaminase capable of converting cytosine or 5-methylcytosine to uracil or thymine, respectively, adenosine deaminase capable of converting adenine to hypoxanthine, guanosine deaminase capable of converting guanine to xanthine and the like. As cytidine deaminase, more preferred is activation-induced cytidine deaminase (hereinafter to be also referred to as AID) which is an enzyme that introduces a mutation into an immunoglobulin gene in the acquired immunity of vertebrata or the like.

While the derivation of deaminase is not particularly limited, for example, PmCDA1 derived from Petromyzon marinus (Petromyzon marinus cytosine deaminase 1) or AID (Activation-induced cytidine deaminase; AICDA) derived from vertebrata (e.g., mammal such as human, swine, bovine, dog, chimpanzee and the like, birds such as chicken and the like, amphibian such as xenopus and the like, fish such as zebrafish, sweetfish, channel catfish and the like) can be used. The base sequence and amino acid sequence of CDS of PmCDA1 are shown in SEQ ID NOs: 1 and 2.

As the nucleic acid sequence-recognizing module in the nucleic acid-modifying enzyme complex of the present invention, a Type I CRISPR-Cas system is specifically used.

As a molecule having a nucleic acid sequence recognition ability, zinc finger (ZF) motif, TAL effector, PPR motif and the like are known. However, the production efficiency of ZF of a ZF motif that specifically binds to the target nucleotide sequence is not high and selection of ZF having high binding specificity is complicated, and therefore, production of a large number of ZF motifs that actually function is not easy. On the other hand, while TAL effector and PPR motif have a high degree of freedom in the target nucleic acid sequence recognition as compared to ZF, since a huge protein according to the target nucleotide sequence needs to be designed and constructed each time, they have a problem in terms of efficiency. In contrast, the CRISPR-Cas system, which is an acquired immunity mechanism widely distributed in eubacteria and archaebacteria, can target any sequence merely by synthesizing an oligoDNA capable of specifically forming a hybrid with the target nucleotide sequence since CRISPR-RNA (crRNA) complementary to the target nucleotide sequence recognize the sequence of the object double stranded DNA.

In the present specification, the "target nucleotide sequence" means a double stranded DNA sequence to which a nucleic acid sequence-recognizing module (specifically, Type I CRISPR-Cas) binds, and a strand that hybridizes to crRNA is referred to as a "targeted strand", and an opposite strand thereof which becomes single-stranded by hybridization to a target strand and crRNA is referred to as a "non-targeted strand". Since a nucleic acid base conversion reaction generally frequently occurs on a single-stranded non-target strand, when the target nucleotide sequence is to be expressed by a single strand (e.g., when PAM sequence is indicated, when positional relationship of target nucleotide sequence and PAM is shown etc.), it is represented by a sequence of a non-target strand.

CRISPR-Cas system is largely divided into three types. One currently frequently used for genome editing is the Type II CRISPR-Cas system in which Cas9 has both a DNA sequence recognition ability and a nuclease activity. On the other hand, in the Type I CRISPR-Cas system, the function is shared between Cas protein complex Cascade, which presents crRNA containing a sequence complementary to the target nucleotide sequence and recognizes the target nucleotide sequence, and Cas3 having nuclease and helicase activities. Therefore, DSB with strong cytotoxicity can be avoided by simply removing Cas3 from the Cas operon even without using a mutant having an inactivated cleavage ability of at least one strand of a double-stranded DNA like Cas9. That is, (a) a DNA encoding a chimeric crRNA in which 5' handle and 3' handle of crRNA are linked to a sequence complementary to a target strand of a target nucleotide sequence (targeting sequence), and (b) a DNA encoding a protein group constituting a Cascade complex and a deaminase are introduced into a host cell having a double stranded DNA to be modified and expressed to form a Cascade complex composed of the crRNA containing the targeting sequence and the protein group in the host cell, whereby the target nucleotide sequence on the double stranded DNA can be identified. The DNA encoding the deaminase is disposed in the DNA of the above-mentioned (b) such that the deaminase here is expressed in a form capable of forming a complex with CasE in the protein group constituting the Cascade. In this way, the deaminase can convert the target nucleotide sequence and the nucleic acid bases in the vicinity thereof recognized and bound by the Cascade complex to other bases.

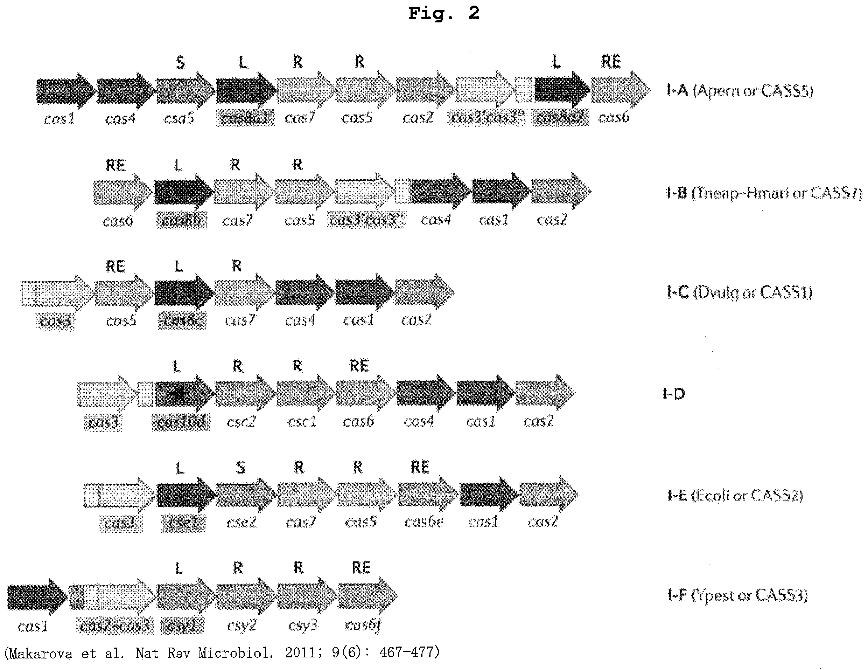

Type I CRISPR-Cas system is known to contain six subtypes of A - F. Fig. 2 schematically shows the Cas operons of subtypes I-A to I-F. In the operon of each subtype, the protein group excluding Cas3 which is nuclease/helicase, Cas1 involved in foreign gene cleavage during immunity acquisition, Cas2 and Cas4 constitutes the Cascade complex. Cse1, Cse2, Cas7, Cas5 and Cas6e in the I-E subtypes are also respectively called CasA, CasB, CasC, CasD and CasE (see Fig. 1; the latter names are used in the present specification). In the I-E subtype, a Cascade complex is formed by CasA, CasB, CasC, CasD and CasE respectively at 1:2:6:1:1 molecule crRNA (see Fig. 1). In the I-E subtype, CasA is considered to play an important role in the identification of PAM sequence (ATG, AAG, AGG or GAG) .

Relatively many I-A, I-B and I-D subtypes are distributed in archaebacterium, and relatively many I-C, I-E and I-F subtypes are distributed in eubacterium. The I-A subtype is analyzed in S. solfataricus, T. tenax and the like, I-B subtype is analyzed in Haloferax volcanii and the like, I-C subtype is analyzed in B. halodurans and the like, I-E subtype is analyzed in Escherichia coli and the like, and I-F subtype is analyzed in P. aeruginosa, Escherichia coli, P. atospeticum and the like.

Therefore, a DNA encoding a protein group constituting a Cascade complex can be obtained by, for example, isolating a region containing ORF of cas5 from csa5 in the case of I-A subtype, a region containing ORF of cas5 from csa6 in the case of I-B subtype, a region containing ORF of cas7 from csa5 in the case of I-C subtype, a region containing ORF of casE from casA in the case of I-E subtype, a region containing ORF of cas6f from csy1 in the case of I-F subtype, from cas operon by genome PCR using genome DNA derived from the above-mentioned bacterial strain as a template. Preferably, the protein groups constituting the Cascade complexes are CasA, CasB, CasC, CasD and CasE constituting Cascade complexes of I-E subtypes, more preferably CasA, CasB, CasC, CasD and CasE derived from Escherichia coli. The ORF sequences of casA, casB, casC, casD and casE derived from Escherichia coli are respectively the 5933rd-7441st, 5458th-5940th, 4341st-5432nd, 3664th-4338th and 3081st-3677th (all Opposite strands) sequences of the nucleotide sequence shown in SEQ ID NO: 4.

A protein group constituting a DNA encoding a Cascade complex can be obtained by chemically synthesizing the DNA strand, or by connecting synthesized partly overlapping oligoDNA short strands by utilizing the PCR method and the Gibson Assembly method to construct a DNA encoding the full length thereof. The advantage of constructing a full-length DNA by chemical synthesis or a combination of PCR method or Gibson Assembly method is that the codon to be used can be designed in CDS full-length according to the host into which the DNA is introduced. In the expression of a heterologous DNA, the protein expression level is expected to increase by converting the DNA sequence thereof to a codon highly frequently used in the host organism. As the data of codon use frequency in host to be used, for example, the genetic code use frequency database (http://www.kazusa.or.jp/codon/index.html) disclosed in the home page of Kazusa DNA Research Institute can be used, or documents showing the codon use frequency in each host may be referred to. By reference to the obtained data and the DNA sequence to be introduced, codons showing low use frequency in the host from among those used for the DNA sequence may be converted to a codon coding the same amino acid and showing high use frequency.

A DNA encoding a deaminase can also be cloned similarly from the enzyme-producing cells. For example, a DNA encoding PmCDA1 of Petromyzon marinus can be cloned by designing suitable primers for the upstream and downstream of CDS based on the cDNA sequence (accession No. EF094822) registered in the NCBI database, and cloning from Petromyzon marinus-derived mRNA by the RT-PCR method. A DNA encoding human AID can be cloned by designing suitable primers for the upstream and downstream of CDS based on the cDNA sequence (accession No. AB040431) registered in the NCBI database, and cloning from, for example, human lymph node-derived mRNA by the RT-PCR method. AID homologues derived from other vertebratas can also be cloned in the same manner as in the above based on known cDNA sequence information (e.g., swine (accession No. CU582981), bovine (accession No. NM_110138682), dog (accession No. NM_001003380), chimpanzee (accession No. NM_001071809), chicken (accession No. NM_001243222), xenopus (accession No. NM_001095712), zebrafish (accession No. AAI62573), sweetfish (accession No. AB619797) and channel catfish (accession No. NM_001200185) etc.).

Alternatively, similar to the above, it can also be constructed as a DNA showing codon usage suitable for expression in a host cell to be used, by a combination of chemical synthesis or PCR method or Gibson Assembly method.

A DNA encoding a cloned or synthesized deaminase may be directly, or after digestion with a restriction enzyme when desired, or after addition of a suitable linker and/or a nuclear localization signal (each oraganelle transfer signal when the object double stranded DNA is mitochondria or chloroplast DNA), linked with a DNA encoding one or more proteins selected from a group of proteins constituting the Cascade complex. While the linker to be used is not particularly limited, for example, Flag-tag, GS linker, Strep-tag and the like can be mentioned, and tandem repeats of these are also encompassed therein.

Alternatively, a DNA encoding a protein constituting the Cascade complex, and a DNA encoding a deaminase may be each fused with a DNA encoding a binding domain (e.g., SH3 domain, PDZ domain, GK domain, GB domain etc.) or a binding partner thereof, or both DNAs may be fused with a DNA encoding a separation intein, whereby the protein constituting the Cascade complex and the deaminase are translated in a host cell to form a complex. Furthermore, a deaminase and a Cascade constituent protein may be bound using an RNA scaffold by RNA aptamer (MS2F6, PP7 etc.) and their binding proteins. Also in these cases, a linker and/or a nuclear localization signal can be linked to a suitable position of one of or both DNAs when desired.

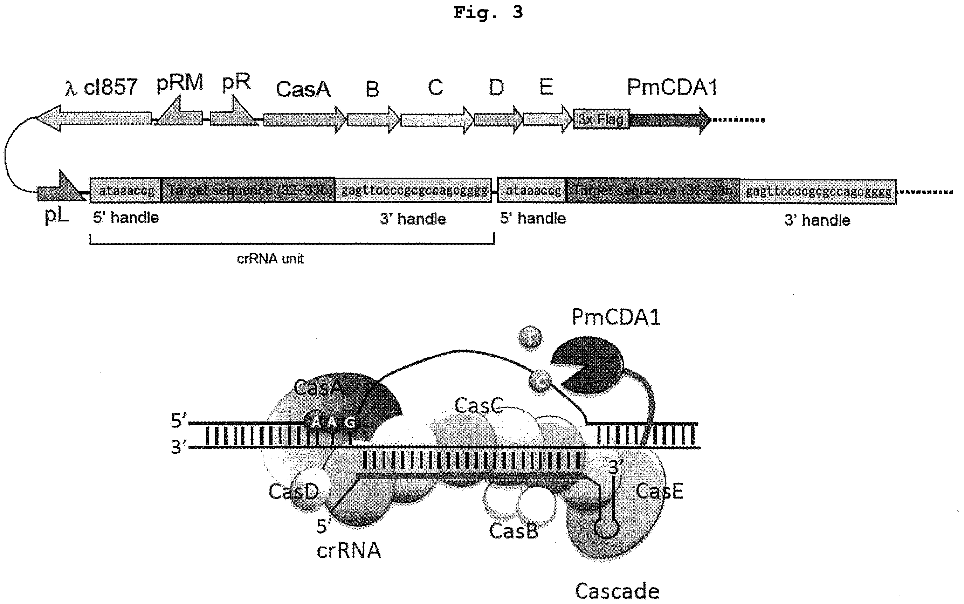

While the Cascade constituent protein that forms a complex with the deaminase is not particularly limited, when it is expressed as a fusion protein, it is easy for gene manipulation to link the protein to the terminal of the operon (downstream of CasE on the C-terminal side in the case of I-E subtype (see Fig. 3).

A complex of a nucleic acid base converting enzyme and a Cascade constituent protein can also be formed using a binding domain and the like by, similarly, linking a DNA encoding a binding domain or a binding partner thereof to the terminal or inside of the operon and linking the binding partner or binding domain to a DNA encoding the nucleic acid base converting enzyme.

The DNA encoding a chimeric crRNA in which 5' handle and 3' handle of crRNA are linked to a sequence complementary to a target strand of a target nucleotide sequence (targeting sequence) can be chemically synthesized by designing an oligo DNA sequence in which known 5' handle and 3' handle (e.g., ataaaccg as 5' handle and gagttccccgcgccagcgggg (SEQ ID NO: 3) as 3' handle when I-E subtype derived from Escherichia coli is used as Cascade complex) are respectively linked to the 5' upstream and 3' downstream of the targeting sequence and using a DNA/RNA synthesizer. The 5' handle and 3' handle described in J. Biol. Chem. 286(24): 21643-21656 (2011) can be used when I-A subtype derived from S. solfataricus is used, the 5' handle and 3' handle described in J. Biol. Chem. 287(40): 33351-33365 (2012) can be used when I-B subtype derived from H. volcanii is used, the 5' handle and 3' handle described in Structure 20: 1574-1584 (2012) can be used when I-C subtype derived from B. halodurans is used, and the 5' handle and 3' handle described in PNAS 108(25): 10092-10097 (2011) can be used when I-F subtype derived from P. aeruginosa is used.

The length of the targeting sequence is not particularly limited as long as it is correctly presented to a Cascade complex and can specifically bind to a target strand of a target nucleotide sequence. It is, for example, 30 - 45 nucleotides, preferably 32 - 33 nucleotides for I-C, I-E and IF subtypes, and preferably 34-44 nucleotides for I-A and I-B subtypes.

The target nucleotide sequence in the Type I CRISPR-Cas system is subject to restriction by PAM sequence specific to the subtype. In the I-E subtype, any of AAG, ATG, AGG, GAG needs to be adjacent to the non-target strand on the 5' upstream side of the target nucleotide sequence in the 5' →3' direction. For example, in the I-E subtype, when a targeting sequence with 32 nucleotide length is used in combination with deaminase, as shown in the below-mentioned Examples, cytosine present at 32 - 44 bases downstream of the PAM sequence causes base conversion, and a mutation is introduced highly frequently.

Since the nucleic acid-modifying enzyme complex of the present invention, in which the Type I CRISPR-Cas system and a deaminase are combined, does not accompany double stranded DNA breaks (DSB), genome editing with low toxicity is possible, and the genetic modification method of the present invention can be applied to a wide range of biological materials. Therefore, the cells into which (a) a DNA encoding a chimeric crRNA in which 5' handle and 3' handle of crRNA are linked to the targeting sequence and (b) a DNA encoding a protein group constituting a Cascade complex and a deaminase are introduced can encompass cells of any species, from bacterium of Escherichia coli and the like which are prokaryotes, cells of microorganism such as yeast which are lower eucaryotes, to cells of vertebrata including mammals such as human (with the proviso that modification of the germ line identity of human beings is excluded), and cells of higher eukaryote such as insect and plant.

An expression vector containing the DNAs of the above-mentioned (a) and (b) can be produced, for example, by linking the DNA to the downstream of a promoter in a suitable expression vector. The DNAs of (a) and (b) may be combined on the same vector or separate vectors.

As the expression vector, Escherichia coli-derived plasmids (e.g., pBR322, pBR325, pUC12, pUC13); Bacillus subtilis-derived plasmids (e.g., pUB110, pTP5, pC194); yeast-derived plasmids (e.g., pSH19, pSH15); insect cell expression plasmids (e.g., pFast-Bac); animal cell expression plasmids (e.g., pA1-11, pXT1, pRc/CMV, pRc/RSV, pcDNAI/Neo); bacteriophages such as λphage; insect virus vectors such as baculovirus (e.g., BmNPV, AcNPV); animal virus vectors such as retrovirus, vaccinia virus, and adenovirus are used.

As the promoter, any promoter appropriate for a host to be used for gene expression can be used. In a conventional method using DSB, since the survival rate of the host cell sometimes decreases markedly due to the toxicity, it is desirable to increase the number of cells by the start of the induction by using an inductive promoter. However, since sufficient cell proliferation can also be afforded by expressing the nucleic acid-modifying enzyme complex of the present invention, a constituent promoter can also be used without limitation.

When the host is Escherichia coli, trp promoter, lac promoter, recA promoter, λP_{L} promoter, λP_{R} promoter, lpp promoter, and T7 promoter are preferable.

When the host is genus Bacillus, SPO1 promoter, SPO2 promoter, and penP promoter are preferable.

When the host is a yeast, Gal1/10 promoter, PHO5 promoter, PGK promoter, GAP promoter, and ADH promoter are preferable.

When the host is an insect cell, polyhedrin promoter, and P10 promoter are preferable.

For example, when the host is an animal cell, SRα promoter, SV40 promoter, LTR promoter, CMV (cytomegalovirus) promoter, RSV (Rous sarcoma virus) promoter, MoMuLV (Moloney mouse leukemia virus) LTR, and HSV-TK (simple herpes virus thymidine kinase) promoter are used. Of these, CMV promoter, and SRα promoter are preferable.

When the host is a plant cell, CaMV35S promoter, CaMV19S promoter, and NOS promoter are preferable.

A DNA encoding the chimeric crRNA can also use a pol III system promoter (e.g., SNR6, SNR52, SCR1, RPR1, U6, H1 promoter etc.) as the promoter and a terminator (e.g., T₆ sequence).

When prokaryotic cell or lower eukaryotic cell such as yeast and the like is used as a host, a DNA encoding a Cascade constituent protein group can be polycistronically expressed as operon (see Fig. 3). When a higher eukaryotic cell such as animal cell and the like is used as a host, an intervening sequence (e.g., IRES sequence, 2A sequence derived from foot- and-mouth disease virus and the like) that enables polycistronic expression can be inserted between respective DNAs encoding Cas proteins. Alternatively, a promoter may be inserted into the 5' upstream of each DNA to cause monocistronic expression.

DNAs encoding the chimeric crRNA can also be inserted in tandem in the downstream of one promoter (see Fig. 3). In this case, the targeting sequences mounted on respective chimeric crRNAs may be the same or different. When the sequences are different, a different region in the same gene may be used as the target nucleotide sequence or a region in a different gene may be used as the target nucleotide sequence. The linking site between respective chimeric crRNA units is a 3' handle-5' handle crRNA repeat sequence having a partial secondary structure of a characteristic hairpin structure. It is therefore considered to be cleaved by a non-specific RNase endogenous to the host cell. The sequence can also be intracellularly cleaved into each chimeric crRNA unit even in a host cell without an endogenous Type I CRISPR-Cas system. It is of course possible to express each chimeric crRNA individually by inserting a promoter in the upstream of the chimeric crRNA.

As the expression vector, besides those mentioned above, one containing enhancer, splicing signal, terminator, polyA addition signal, a selection marker such as drug resistance gene, auxotrophic complementary gene and the like, replication origin and the like on demand can be used.

A nucleic acid-modifying enzyme complex of the present invention can be intracellularly expressed by introducing an expression vector containing (a) a DNA encoding chimeric crRNA and (b) a DNA encoding a Cascade constituent protein group and a deaminase into a host cell, and culturing the host cell.

As the host, genus Escherichia, genus Bacillus, yeast, insect cell, insect, animal cell and the like are used.

As the genus Escherichia, Escherichia coli K12-DH1 [Proc. Natl. Acad. Sci. USA, 60, 160 (1968)], Escherichia coli JM103 [Nucleic Acids Research, 9, 309 (1981)], Escherichia coli JA221 [Journal of Molecular Biology, 120, 517 (1978)], Escherichia coli HB101 [Journal of Molecular Biology, 41, 459 (1969)], Escherichia coli C600 [Genetics, 39, 440 (1954)] and the like are used.

As the genus Bacillus, Bacillus subtilis MI114 [Gene, 24, 255 (1983)], Bacillus subtilis 207-21 [Journal of Biochemistry, 95, 87 (1984)] and the like are used.

As a bacterium having an endogenous Type I CRISPR-Cas system, a mutant strain deficient in endogenous Cas3 is desirably used.

As the yeast, Saccharomyces cerevisiae AH22, AH22R⁻, NA87-11A, DKD-5D, 20B-12, Schizosaccharomyces pombe NCYC1913, NCYC2036, Pichia pastoris KM71 and the like are used.

As the insect cell when the virus is AcNPV, cells of cabbage armyworm larva-derived established line (Spodoptera frugiperda cell; Sf cell), MG1 cells derived from the mid-intestine of Trichoplusia ni, High Five^{™} cells derived from an egg of Trichoplusia ni, Mamestra brassicae-derived cells, Estigmena acrea-derived cells and the like are used. When the virus is BmNPV, cells of Bombyx mori-derived established line (Bombyx mori N cell; BmN cell) and the like are used as insecT cells. As the Sf cell, for example, Sf9 cell (ATCC CRL1711), Sf21 cell [all above, In Vivo, 13, 213-217 (1977)] and the like are used.

As the insect, for example, larva of Bombyx mori, Drosophila, cricket and the like are used [Nature, 315, 592 (1985)].

As the animal cell, cell lines such as monkey COS-7 cell, monkey Vero cell, Chinese hamster ovary (CHO) cell, dhfr gene-deficient CHO cell, mouse L cell, mouse AtT-20 cell, mouse myeloma cell, rat GH3 cell, human FL cell, pluripotent stem cells such as iPS cell, ES cell of human and other mammals, and primary cultured cells prepared from various tissues are used (with the proviso that modification of the human germ line identity of human beings is excluded). Furthermore, zebrafish embryo and Xenopus oocyte can also be used.

As the plant cell, suspend cultured cells, callus, protoplast, leaf segment, root segment and the like prepared from various plants (e.g., grain such as rice, wheat, corn and the like, product crops such as tomato, cucumber, egg plant and the like, garden plants such as carnation, Eustoma russellianum and the like, experiment plants such as tobacco, arabidopsis thaliana and the like, and the like) are used.

All the above-mentioned host cells may be haploid (monoploid), or polyploid (e.g., diploid, triploid, tetraploid and the like). In the conventional mutation introduction methods, mutation is, in principle, introduced into only one homologous chromosome to produce a hetero gene type. Therefore, desired phenotype is not expressed unless dominant mutation occurs, and homozygousness inconveniently requires labor and time. In contrast, according to the present invention, since mutation can be introduced into any allele on the homologous chromosome in the genome, desired phenotype may be expressed in a single generation even in the case of recessive mutation, and the problems of the conventional methods can be solved.

An expression vector can be introduced by a known method (e.g., lysozyme method, competent method, PEG method, CaCl₂ coprecipitation method, electroporation method, the microinjection method, the particle gun method, lipofection method, Agrobacterium method and the like) according to the kind of the host.

Escherichia coli can be transformed according to the methods described in, for example, Proc. Natl. Acad. Sci. USA, 69, 2110 (1972), Gene, 17, 107 (1982) and the like.

A vector can be introduced into the genus Bacillus according to the methods described in, for example, Molecular & General Genetics, 168, 111 (1979) and the like.

A vector can be introduced into a yeast according to the methods described in, for example, Methods in Enzymology, 194, 182-187 (1991), Proc. Natl. Acad. Sci. USA, 75, 1929 (1978) and the like.

A vector can be introduced into an insect cell and insect according to the methods described in, for example, Bio/Technology, 6, 47-55 (1988) and the like.

A vector can be introduced into an animal cell according to the methods described in, for example, Cell Engineering additional volume 8, New Cell Engineering Experiment Protocol, 263-267 (1995) (published by Shujunsha), and Virology, 52, 456 (1973) .

A cell introduced with a vector can be cultured according to a known method according to the kind of the host.

For example, when Escherichia coli or genus Bacillus is cultured, a liquid medium is preferable as a medium to be used for the culture. The medium preferably contains a carbon source, nitrogen source, inorganic substance and the like necessary for the growth of the transformant. Examples of the carbon source include glucose, dextrin, soluble starch, sucrose and the like; examples of the nitrogen source include inorganic or organic substances such as ammonium salts, nitrate salts, corn steep liquor, peptone, casein, meat extract, soybean cake, potato extract and the like; and examples of the inorganic substance include calcium chloride, sodium dihydrogen phosphate, magnesium chloride and the like. The medium may contain yeast extract, vitamins, growth promoting factor and the like. The pH of the medium is preferably about 5 - about 8.

As a medium for culturing Escherichia coli, for example, M9 medium containing glucose, casamino acid [Journal of Experiments in Molecular Genetics, 431-433, Cold Spring Harbor Laboratory, New York 1972] is preferable. Where necessary, for example, agents such as 3β-indolylacrylic acid may be added to the medium to ensure an efficient function of a promoter. Escherichia coli is cultured at generally about 15 - about 43°C. Where necessary, aeration and stirring may be performed.

The genus Bacillus is cultured at generally about 30 - about 40°C. Where necessary, aeration and stirring may be performed.

Examples of the medium for culturing yeast include Burkholder minimum medium [Proc. Natl. Acad. Sci. USA, 77, 4505 (1980)], SD medium containing 0.5% casamino acid [Proc. Natl. Acad. Sci. USA, 81, 5330 (1984)] and the like. The pH of the medium is preferably about 5 - about 8. The culture is performed at generally about 20°C - about 35°C. Where necessary, aeration and stirring may be performed.

As a medium for culturing an insect cell or insect, for example, Grace's Insect Medium [Nature, 195, 788 (1962)] containing an additive such as inactivated 10% bovine serum and the like as appropriate and the like are used. The pH of the medium is preferably about 6.2 - about 6.4. The culture is performed at generally about 27°C. Where necessary, aeration and stirring may be performed.

As a medium for culturing an animal cell, for example, minimum essential medium (MEM) containing about 5 - about 20% of fetal bovine serum [Science, 122, 501 (1952)], Dulbecco's modified Eagle medium (DMEM) [Virology, 8, 396 (1959)], RPMI 1640 medium [The Journal of the American Medical Association, 199, 519 (1967)], 199 medium [Proceeding of the Society for the Biological Medicine, 73, 1 (1950)] and the like are used. The pH of the medium is preferably about 6 - about 8. The culture is performed at generally about 30°C - about 40°C. Where necessary, aeration and stirring may be performed.

As a medium for culturing a plant cell, for example, MS medium, LS medium, B5 medium and the like are used. The pH of the medium is preferably about 5 - about 8. The culture is performed at generally about 20°C - about 30°C. Where necessary, aeration and stirring may be performed.

As mentioned above, a complex of a nucleic acid sequence-recognizing module (Type I CRISPR-Cas) and a deaminase, i.e., nucleic acid-modifying enzyme complex, can be expressed intracellularly.

When (a) a chimeric crRNA and (b) a complex of a Cascade constituent protein group and a deaminase are expressed from an expression vector introduced into a cell, the protein group forms a Cascade complex and presents the chimeric crRNA. When the Cascade complex specifically recognizes and binds to a target nucleotide sequence in the double stranded DNA (e.g., genomic DNA) of interest, due to the action of the deaminase linked to the Cascade constituent protein group, base conversion occurs in the sense strand or antisense strand of the targeted site (whole or partial target nucleotide sequence or vicinity thereof) and a mismatch occurs in the double stranded DNA (e.g., when cytidine deaminase such as PmCDA1, AID and the like is used as a deaminase, cytosine on the sense strand or antisense strand at the targeted site is converted to uracil to cause U:G or G:U mismatch). When the mismatch is not correctly repaired, and when repaired such that a base of the opposite strand forms a pair with a base of the converted strand (T-A or A-T in the above-mentioned example), or when other nucleotide is further substituted (e.g., U→A, G) or when one to several dozen bases are deleted or inserted during repair, various mutations are introduced.

Since conventional artificial nuclease accompanies double stranded DNA breaks (DSB), inhibition of growth and cell death assumedly caused by disordered cleavage of chromosome (off-target cleavage) occur by targeting a sequence in the genome. The effect thereof is particularly fatal for many microorganisms and prokaryotes, and prevents applicability. In the present invention, mutation is introduced not by DNA cleavage but by a substituent conversion reaction (particularly deamination reaction) on the DNA base, and therefore, drastic reduction of toxicity can be realized.

The modification of the double stranded DNA in the present invention does not prevent occurrence of cleavage of the double stranded DNA in a site other than the targeted site. However, one of the greatest advantages of the present invention is avoidance of toxicity by off-target cleavage, which is generally applicable to any species. In preferable one embodiment, therefore, the modification of the double stranded DNA in the present invention does not accompany cleavage of DNA strand not only in a targeted site of a given double stranded DNA but in a site other than same.

The present inventors have already found that, when Type II CRIPR-Cas system is used as a nucleic acid sequence-recognizing module and AID is used as a deaminase, it is preferable to design the target nucleotide sequence such that C (or G in the opposite strand) into which a mutation is desired to be introduced is at 2 - 5 nucleotides from the 5'-end of the target nucleotide sequence. In the Type II CRISPR-Cas system, the length of the targeting sequence can be appropriately determined to fall between 15 - 30 nucleotides, preferably 18 - 25 nucleotides. Since the targeting sequence is a sequence complementary to the target strand of the target nucleotide sequence, the length of the target nucleotide sequence changes when the length of the targeting sequence is changed; however, the regularity that a mutation is easily introduced into C or G at 2 - 5 nucleotides from the 5'-end irrespective of the length of the nucleotide is maintained. Therefore, by appropriately determining the length of the target nucleotide sequence (targeting sequence as a complementary strand thereof), the site of a base into which a mutation can be introduced can be shifted. As a result, restriction by PAM (NGG in the case of SpCas9) can be removed partially but not completely. On the other hand, when the Type I-E CRISPR-Cas system (targeting sequence 32 nucleotides) was used as a nucleic acid sequence-recognizing module and AID was used as a deaminase, a mutation was mainly introduced into the range of 32 - 44 bases downstream of PAM (AAG, ATG, AGG, GAG in I-E subtype derived from Escherichia coli). As mentioned above, the PAM sequence varies according to the Type I subtype and has a high degree of freedom of 2 - 3 nucleotides (e.g., CCN in I-A subtype, CC in I-F subtype). In addition, it shows highly frequent mutation introduction in a wider range than by the use of the Type II CRISPR-Cas system. While the tolerance of the length of the targeting sequence has a room for consideration, a longer target nucleotide sequence can be determined by using the I-A or I-B subtype.

By utilizing the Type I CRISPR-Cas system as a nucleic acid sequence-recognizing module in this manner, a mutation can be introduced even into a site difficult for mutation introduction in the Type II CRISPR-Cas system, and the systems can be utilized complementarily.

As shown in Fig. 3, a chimeric crRNA containing multiple targeting sequences can be introduced into a host cell. The present inventors have already found that, when Type II CRISPR-Cas system is used and sequence-recognizing modules are produced corresponding to the adjacent multiple target nucleotide sequences and simultaneously used, the mutation introduction efficiency markedly increases than by using a single nucleotide sequence as a target. As the effect thereof, similarly mutation induction is realized even when both target nucleotide sequences partly overlap or when the both are apart by about 600 bp. mutation introduction can occur both when the target nucleotide sequences are in the same direction (target strands are present on the same strand), and when they are opposed (target strand is present on each strand of double stranded DNA).

Therefore, further improvement of the mutation induction efficiency can be expected in the present invention using the Type I CRISPR-Cas system by targeting multiple nucleotide sequences.

To express the nucleic acid-modifying enzyme complex of the present invention in a host cell, as mentioned above, an expression vector containing a DNA encoding the nucleic acid-modifying enzyme complex is introduced into a host cell. For efficient introduction of mutation, it is desirable to maintain an expression of nucleic acid-modifying enzyme complex of a given level or above for not less than a given period. From such aspect, it is ensuring to introduce an expression vector (plasmid etc.) autonomously replicatable in a host cell. However, since the plasmid etc. are foreign DNAs, they are preferably removed rapidly after successful introduction of mutation. Therefore, though subject to change depending on the kind of host cell and the like, for example, the introduced plasmid is desirably removed from the host cell after a lapse of 6 hr - 2 days from the introduction of an expression vector by using various plasmid removal methods well known in the art.

Alternatively, as long as expression of a nucleic acid-modifying enzyme complex, which is sufficient for the introduction of mutation, is obtained, it is preferable to introduce mutation into the object double stranded DNA by transient expression by using an expression vector without autonomous replicatability in a host cell (e.g., vector etc. lacking replication origin that functions in host cell and/or gene encoding protein necessary for replication).

Expression of target gene is suppressed while the nucleic acid-modifying enzyme complex of the present invention is expressed in a host cell to perform a nucleic acid base conversion reaction. Therefore, it was difficult to directly edit a gene essential for the survival of the host cell as a target gene (due to side effects such as growth inhibition of host, unstable mutation introduction efficiency, mutation of site different from target and the like). In the present invention, direct editing of an essential gene is successfully realized by causing a nucleic acid base conversion reaction in a desired stage, and transiently expressing the nucleic acid-modifying enzyme complex of the present invention in a host cell for a period necessary for fixing the modification of the targeted site. While a period necessary for a nucleic acid base conversion reaction and fixing the modification of the targeted site varies depending on the kind of the host cell, culture conditions and the like, 2 - 20 generations are generally considered to be necessary. For example, when the host cell is yeast or bacterium (e.g., Escherichia coli), expression of a nucleic acid-modifying enzyme complex needs to be induced for 5 - 10 generations. Those of ordinary skill in the art can appropriately determine a preferable expression induction period based on the doubling time of the host cell under culture conditions to be used. The expression induction period of the a nucleic acid encoding the nucleic acid-modifying enzyme complex of the present invention may be extended beyond the above-mentioned "period necessary for fixing the modification of the targeted site" as long as the host cell is free of side effects.

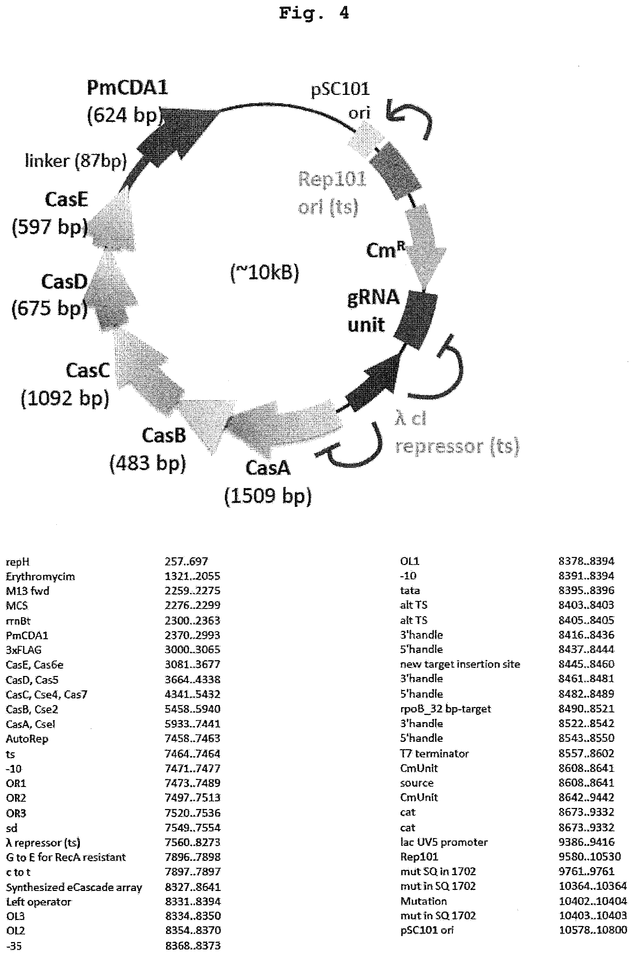

As a means for transiently expressing the nucleic acid-modifying enzyme complex of the present invention at a desired stage for a desired period, a method including producing a construct (expression vector) containing a DNA encoding the nucleic acid-modifying enzyme complex, in a form capable of controlling the expression period, introducing the construct into a host cell can be mentioned. The "form capable of controlling the expression period" is specifically, for example, a DNA encoding the nucleic acid-modifying enzyme complex of the present invention placed under regulation of an inducible regulatory region. While the "inducible regulatory region" is not particularly limited, it is, for example, in microbial cells of bacterium (e.g., Escherichia coli), yeast and the like, an operon of a temperature sensitive (ts) mutation repressor and an operator regulated thereby (see Fig. 4). Examples of the ts mutation repressor include, but are not limited to, ts mutation of λphage-derived cI repressor. In the case of λphage cI repressor (ts), it is bound to an operator to suppress expression of gene in the downstream at not more than 30°C (e.g., 28°C). At a high temperature of not less than 37°C (e.g., 42°C), it is dissociated from the operator to allow for induction of gene expression. Therefore, the period when the expression of the target gene is suppressed can be minimized by culturing a host cell introduced with a DNA encoding nucleic acid-modifying enzyme complex generally at not more than 30°C, raising the temperature to not less than 37°C at an appropriate stage, performing culture for a given period to carry out a nucleic acid base conversion reaction and, after introduction of mutation into the target gene, rapidly lowering the temperature to not more than 30°C. Thus, even when an essential gene for the host cell is targeted, it can be efficiently edited while suppressing the side effects.

When temperature sensitive mutation is utilized, for example, a temperature sensitive mutant of a protein necessary for autonomous replication of a vector is mounted on a vector containing a DNA encoding the nucleic acid-modifying enzyme complex of the present invention. As a result, autonomous replication cannot occur rapidly after expression of the nucleic acid-modifying enzyme complex, and the vector naturally falls off along with the cell division. Examples of the temperature sensitive mutant protein include, but are not limited to, a temperature sensitive mutant of Rep101 ori necessary for the replication of pSC101 ori (see Fig. 4). Rep101 ori (ts) acts on pSC101 ori to enable autonomous replication of plasmid at not more than 30°C (e.g., 28°C), but loses function at not less than 37°C (e.g., 42°C), and plasmid cannot replicate autonomously. Therefore, a combined use with cI repressor (ts) of the above-mentioned λphage simultaneously enables transient expression of the nucleic acid-modifying enzyme complex of the present invention, and removal of the plasmid.

On the other hand, when a higher eukaryotic cell such as animal cell, insect cell, plant cell and the like is used as a host cell, a DNA encoding the nucleic acid-modifying enzyme complex of the present invention is introduced into a host cell under regulation of an inductive promoter (e.g., metallothionein promoter (induced by heavy metal ion), heat shock protein promoter (induced by heat shock), Tet-ON/Tet-OFF system promoter (induced by addition or removal of tetracycline or a derivative thereof), steroid-responsive promoter (induced by steroid hormone or a derivative thereof) etc.), the induction substance is added to the medium (or removed from the medium) at an appropriate stage to induce expression of the nucleic acid-modifying enzyme complex, culture is performed for a given period to carry out a nucleic acid base conversion reaction and, introduction of mutation into the target gene, transient expression of the nucleic acid-modifying enzyme complex can be realized by removing (or re-adding) the induction substances.

Prokaryotic cells such as Escherichia coli and the like can utilize an inductive promoter. Examples of the inductive promoter include, but are not limited to, lac promoter (induced by IPTG), cspA promoter (induced by cold shock), araBAD promoter (induced by arabinose) and the like.

Alternatively, the above-mentioned inductive promoter can also be utilized as a vector removal mechanism when higher eukaryotic cells such as animal cell, insect cell, plant cell and the like are used as a host cell. That is, a vector is mounted with a replication origin that functions in a host cell, and a nucleic acid encoding a protein necessary for replication (e.g., SV40 ori and large T antigen, oriP and EBNA-1 etc. for animal cells), of the expression of the nucleic acid encoding the protein is regulated by the above-mentioned inductive promoter. As a result, while the vector is autonomously replicatable in the presence of an induction substance, when the induction substance is removed, autonomous replication is not available, and the vector naturally falls off along with cell division (autonomous replication is not possible by the addition of tetracycline and doxycycline in Tet-OFF system vector).

The present invention is explained in the following by referring to Examples, which are not to be construed as limitative.

### [Examples]

### (1) Production of CRISPR/Cascade-PmCDA1 expression vector

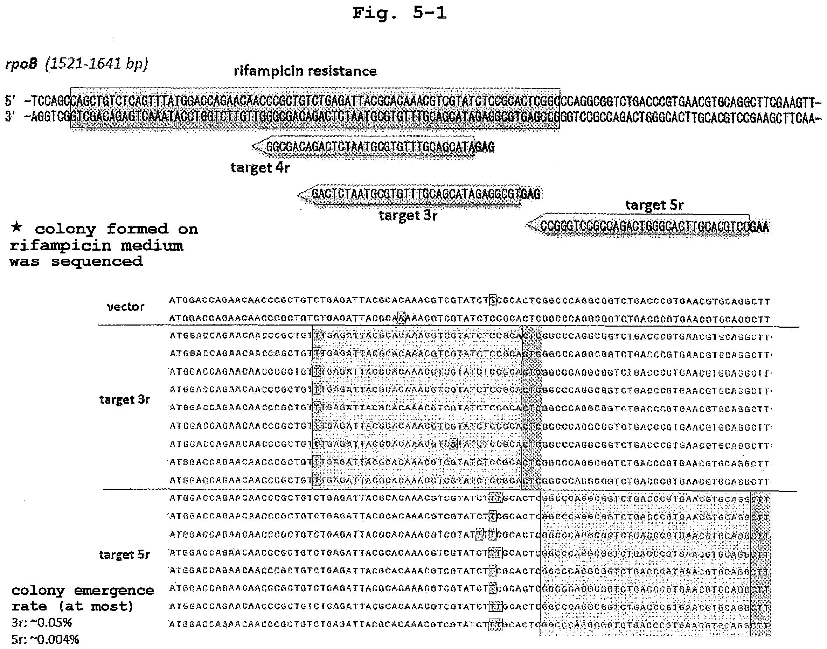

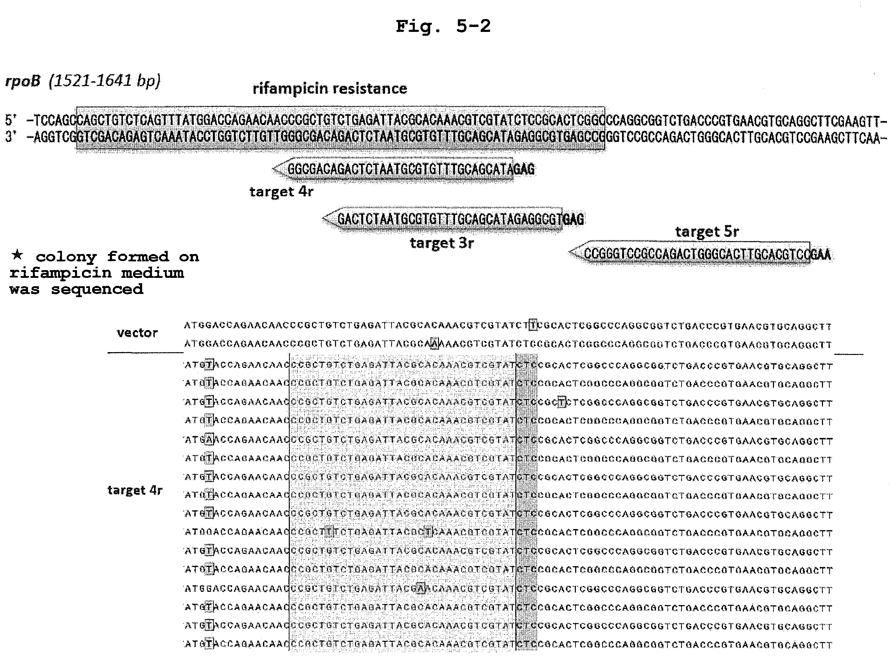

In the Cascade region derived from Escherichia coli DL21 (DE3) strain genome, an operon containing ORF of from CasA to CasE was isolated by PCR, and a composite gene segment in which PmCDA1 gene was fused in the downstream of CasE with 3xFlag tag as a linker was introduced into the downstream of the pR promoter of a temperature induction type vector. As a crRNA region, pL as a promoter and 32bp targeting sequence combined between 5' handle sequence and 3' handle sequence in the downstream of the promoter (see Fig. 5) were introduced. The full-length DNA sequence of the obtained expression vector is shown in SEQ ID NO: 4, and a schematic drawing and the sequence information of the vector are shown in Fig. 4. The targeting sequence is inserted into the portion of n₃₂ in SEQ ID NO: 4.

### (2) Modification of rpoB gene using CRISPR/Cascade-PmCDA1

Nuclease cas3 deficient Escherichia coli strain (JW2731) was used for a genome editing test. Escherichia coli competent cells were produced by a conventional method and transformed with the expression vector produced in the above-mentioned (1). After recovery culture (about 2.5 hr) in SOC medium (500 µl), the cells were diluted with a drug selection medium (LB+10 ug/ml chloramphenicol (Cm)) (2.5 ml) and cultured at a non-induction temperature of 28°C almost one night. Thereafter, the cells were diluted 20-fold with the same medium and shake cultured at 37°C for about 4 hr to induce expression. A 10-fold dilution series of the culture medium was produced, the cells were selected in a Cm-containing or not containing mutation selection plate medium (LB+25 ug/ml rifampicin (Rif)) and Rif resistant colony was obtained.

The target region in the rpoB gene in the obtained colony was amplified by PCR and mutation was identified by sanger sequence. The results are shown in Fig. 5-1 and 5-2. When the target nucleotide sequence was set to 32 nucleotides, cytosine present in 32 - 44 bases downstream from PAM sequence was mainly converted.

### [Industrial Applicability]

According to the present invention, a site specific mutation can be safely introduced into any species without accompanying insertion of foreign DNA or DNA double strand cleavage. Therefore, the present invention is useful for molecular breeding of useful microorganisms.

## Claims

1. A non-therapeutic method of modifying a targeted site of a double stranded DNA in a host cell, the method comprising introducing
(a) a DNA encoding a crRNA comprising a sequence complementary to a target strand of a target nucleotide sequence in the given double stranded DNA, and
(b) a DNA encoding a protein group constituting Cascade and a deaminase, in which the deaminase is capable of forming a complex with CasE in the protein group into the host cell to convert one or more nucleotides in the targeted site to other one or more nucleotides without cleaving the double stranded DNA in the targeted site,
wherein the complex lacks Cas3, Cas1, Cas2 and Cas4, and
wherein the protein group constituting Cascade comprises CasA, CasB, CasC, CasD and CasE, and
wherein the method is not a process for modifying the germ line genetic identity of human beings.

2. The method according to claim 1, wherein the Cascade is derived from Escherichia coli.

3. The method according to claim 1 or 2, wherein the deaminase is cytidine deaminase.

4. The method according to any one of claims 1 to 3, wherein the host cell is a prokaryotic cell.

5. The method according to any one of claims 1 to 4, comprising a step of introducing an expression vector comprising the DNAs of the (a) and (b) in a form capable of controlling an expression period into the host cell, and inducing expression of the DNAs for a period necessary for fixing the modification of the targeted site in the double stranded DNA.

6. The method according to claim 5, wherein the target nucleotide sequence in the double stranded DNA is present in a gene essential for the host cell.

7. A nucleic acid-modifying enzyme complex for modifying a targeted site of a double stranded DNA in a host cell, the complex comprising
(a) a crRNA comprising a sequence complementary to a target strand of a target nucleotide sequence in the given double stranded DNA, and
(b) a nucleic acid-modifying enzyme complex comprising a protein group constituting Cascade, and a deaminase that has formed a complex with CasE in the protein group,
wherein the complex lacks Cas3, Cas1, Cas2 and Cas4, and
wherein the protein group constituting Cascade comprises CasA, CasB, CasC, CasD and CasE.

8. The nucleic acid-modifying enzyme complex according to claim 7, wherein the Cascade is derived from Escherichia coli.

9. The nucleic acid-modifying enzyme complex according to claim 7 or 8, wherein the deaminase is cytidine deaminase.

10. A DNA encoding the nucleic acid-modifying enzyme complex according to any one of claims 7 to 9.

## Patentansprüche

1. Nicht-therapeutisches Verfahren zum Modifizieren einer zielgerichteten Stelle einer doppelsträngigen DNA in einer Wirtszelle, wobei das Verfahren das Einführen umfasst von:
(a) einer DNA, die für eine crRNA kodiert, die eine Sequenz umfasst, die komplementär zu einem Zielstrang einer Zielnukleotidsequenz in der gegebenen doppelsträngigen DNA ist, und
(b) einer DNA, die für eine Proteingruppe, die eine Kaskade bildet, und eine Deaminase kodiert, in der die Deaminase in der Lage ist, einen Komplex mit CasE in der Proteingruppe in die Wirtszelle auszubilden, um ein oder mehrere Nukleotide an der zielgerichteten Stelle in andere ein oder mehrere Nukleotide umzuwandeln, ohne die doppelsträngige DNA in der zielgerichteten Stelle zu spalten,
wobei dem Komplex Cas3, Cas1, Cas2 und Cas4 fehlen, und
wobei die Proteingruppe, die die Kaskade ausbildet, CasA, CasB, Cas, CasD und CasE umfasst, und
wobei das Verfahren kein Vorgang zum Modifizieren der genetischen Identität der Keimbahn des Menschen ist.

2. Verfahren nach Anspruch 1, wobei die Kaskade von Escherichia coli abgeleitet ist.

3. Verfahren nach Anspruch 1 oder 2, wobei die Deaminase Cytidin-Desaminase ist.

4. Verfahren nach einem der Ansprüche 1 bis 3, wobei die Wirtszelle eine prokaryotische Zelle ist.

5. Verfahren nach einem der Ansprüche 1 bis 4, umfassend einen Schritt des Einführens eines Expressionsvektors, der die DNAs der (a) und (b) in einer Form umfasst, die in der Lage ist, eine Expressionsperiode in die Wirtszelle zu steuern, und des Induzierens der Expression der DNAs für eine Periode, die notwendig ist, um die Modifikation der zielgerichteten Stelle in der doppelsträngigen DNA zu fixieren.

6. Verfahren nach Anspruch 5, wobei die Zielnukleotidsequenz in der doppelsträngigen DNA in einem für die Wirtszelle essenziellen Gen vorhanden ist.

7. Nukleinsäure-modifizierender Enzymkomplex zum Modifizieren einer zielgerichteten Stelle einer doppelsträngigen DNA in einer Wirtszelle, wobei der Komplex umfasst
(a) eine crRNA, die eine Sequenz umfasst, die komplementär zu einem Zielstrang einer Zielnukleotidsequenz in der gegebenen doppelsträngigen DNA ist, und
(b) einen Nukleinsäure-modifizierenden Enzymkomplex, der eine Proteingruppe, die Kaskade bildet, und eine Deaminase umfasst, die einen Komplex mit CasE in der Proteingruppe ausgebildet hat,
wobei dem Komplex Cas3, Cas1, Cas2 und Cas4 fehlen, und
wobei die Proteingruppe, die die Kaskade bildet, CasA, CasB, Cas, CasD und CasE umfasst.

8. Nukleinsäure-modifizierender Enzymkomplex nach Anspruch 7, wobei die Kaskade von Escherichia coli abgeleitet ist.

9. Nukleinsäure-modifizierender Enzymkomplex nach Anspruch 7 oder 8, wobei die Deaminase Cytidin-Desaminase ist.

10. DNA, die für den Nukleinsäure-modifizierenden Enzymkomplex nach einem der Ansprüche 7 bis 9 kodiert.

## Revendications

1. Méthode non thérapeutique de modification d'un site ciblé d'ADN à double brin dans une cellule hôte, la méthode comprenant l'introduction de
(a) un ADN codant pour un ARNcr comprenant une séquence complémentaire à un brin cible d'une séquence nucléotidique cible dans l'ADN à double brin donné, et
(b) un ADN codant pour un groupe protéique constituant une cascade et une désaminase, dans lequel la désaminase peut former un complexe avec CasE dans le groupe protéique dans la cellule hôte pour convertir un ou plusieurs nucléotides dans le site ciblé en un ou plusieurs autres nucléotides sans cliver l'ADN à double brin dans le site ciblé,
dans lequel le complexe ne possède pas Cas3, Cas1, Cas2 et Cas4, et
dans lequel le groupe protéique constituant la Cascade comprend CasA, CasB, CasC, CasD et CasE, et
dans laquelle la méthode n'est pas un procédé de modification de l'identité génétique de lignée germinale des êtres humains.

2. Méthode selon la revendication 1, dans laquelle la Cascade est dérivée d'Escherichia coli.

3. Méthode selon la revendication 1 ou 2, dans laquelle la désaminase est la cytidine désaminase.

4. Méthode selon l'une quelconque des revendications 1 à 3, dans laquelle la cellule hôte est une cellule procaryote.

5. Méthode selon l'une quelconque des revendications 1 à 4, comprenant une étape d'introduction d'un vecteur d'expression comprenant les ADN de (a) et (b) sous une forme pouvant contrôler une période d'expression dans la cellule hôte, et d'induction de l'expression des ADN pendant une période nécessaire à la fixation de la modification du site ciblé dans l'ADN à double brin.

6. Méthode selon la revendication 5, dans laquelle la séquence nucléotidique cible dans l'ADN à double brin est présente dans un gène essentiel à la cellule hôte.

7. Complexe enzymatique modificateur d'acide nucléique pour la modification d'un site ciblé d'un ADN à double brin dans une cellule hôte, la cellule comprenant
(a) un ARNcr comprenant une séquence complémentaire à un brin cible d'une séquence nucléotidique cible dans l'ADN à double brin donné, et
(b) un complexe enzymatique modificateur d'acide nucléique comprenant un groupe protéique constituant la Cascade, et une désaminase qui a formé un complexe avec CasE dans le groupe protéique,
dans lequel le complexe ne possède pas Cas3, Cas1, Cas2 et Cas4, et
dans lequel le groupe protéique constituant la Cascade comprend CasA, CasB, CasC, CasD et CasE.

8. Complexe enzymatique modificateur d'acide nucléique selon la revendication 7, dans lequel la Cascade est dérivée d'Escherichia coli.

9. Complexe enzymatique modificateur d'acide nucléique selon la revendication 7 ou 8, dans lequel la désaminase est la cytidine désaminase.

10. ADN codant pour le complexe enzymatique modificateur d'acide nucléique selon l'une quelconque des revendications 7 à 9.
